# EUROPEAN PATENT SPECIFICATION

(11) **EP 0 668 359 B1**
(45) Date of publication and mention of the grant of the patent: **31.05.2000**
(21) Application number: 94102622.1
(22) Date of filing: 22.02.1994
(51) Int. Cl.: C12P 7/52, C12P 19/42

(54) **Continuous fermentation process which is useful for the simultaneous optimal production of propionic acid and vitamin B12**
Kontinuierliches Fermentationsverfahren für die optimale gleichzeitige Herstellung von Propionsäure und Vitamin B12
Procédé de fermentation continu pour la production optimale simultanée d'acide propionique et de vitamine B12

(43) Date of publication of application: 23.08.1995
(73) Proprietor: Gesellschaft für Biotechnologische Forschung mbH (GBF), D-38124 Braunschweig (DE)
(72) Inventor: Scharif-Afschar, Abbas, D-38116 Braunschweig (DE); Quesada-Chanto, Adolfo, D-38300 Wolfenbüttel (DE)
(74) Representative: Savina, Jacques

(56) References cited:
- EP-A- 0 087 920
- PATENT ABSTRACTS OF JAPAN vol. 7, no. 263 (C-196)24 November 1983 & JP-A-58 146 292 (NIPPON SEKIYU KK) 31 August 1983
- BIOTECHNOLOGY LETTERS vol. 11, no. 3 , 1989 pages 189 - 194 P. BLANC ET AL 'Propionic acid and biomass production using continuous ultrafitration fermentation of whey'
- CHEMICAL ABSTRACTS, vol. 72, no. 25, 22 June 1970, Columbus, Ohio, US; abstract no. 131083p, M. NISHIKAWA ET AL 'Fermentation of spent sulfite liquor to produce acetic acid , propionic acid and vitamin B12' page 221 ;column R ; & PULP PAP. MAG. CAN. vol. 71, no. 3 , 1970 pages T59 - T64
- CHEMICAL ABSTRACTS, vol. 85, no. 19, 8 November 1976, Columbus, Ohio, US; abstract no. 141267j, M. BESHKOV ET AL 'Effect of the quantity of carbon source on the biosynthesis of propionic acid and vitamin B12 during propionic acid fermentation with Propionibacterium shermanii' page 388 ;column L ; & NAUCHNI TR., VISSH. INST. KHRANIT. VKUSOVA PROM- ST., PLOVDIV vol. 21, no. 1 , 1974 pages 159 - 167

## Description

The present invention relates to a continuous fermentation process which is suitable for the simultaneous and optimized production of vitamin B₁₂ and propionic acid.

Propionic acid and vitamin B₁₂ are two compounds involved in a large number of industrial operations.

As main outlets for propionic acid, there may be mentioned, in particular, the food industry, in which it is employed as a fungicide in the form of calcium and sodium propionates, the cellulose-based plastics industry and the perfumery sector.

Vitamin B₁₂, for its part, is an important cofactor in the metabolism of carbohydrates, lipids, amino acids and nucleic acids. Vitamin B₁₂ is, moreover, a therapeutic agent used in chemotherapy.

Generally speaking, vitamin B₁₂ is prepared by fermentation. The two main corresponding genera of microorganisms employed for its preparation at industrial level are Propionibacterium and Pseudomonas.

It is noted that, in the standard techniques of production of vitamin B₁₂ using microorganisms of the genus Propionibacterium, the growth of these latter becomes impaired during the fermentation process, leading to a fall in productivity with respect to vitamin B₁₂. This is the direct consequence of the concomitant formation of propionic acid in the culture medium. The amount of propionic acid increases during the fermentation process, and reaches a certain limit which inhibits the growth of the said microorganisms.

Traditionally, the industrial production of propionic acid is chiefly carried out by petrochemical methods.

Production by fermentation also proves possible, but is not satisfactory from an industrial standpoint. In general, it employs the assimilation of glucose by propionibacteria and leads to the formation of propionic acid but also of not insignificant amounts of acetic acid. Lastly, according to this fermentation process, low yields of propionic acid are obtained on account of the phenomenon already described above in the case of vitamin B₁₂ production, namely an inhibition of the growth of the Propionibacterium bacteria by propionic acid.

The purpose of the present invention is to propose a production process common to vitamin B₁₂ and propionic acid.

This process employs the fermentation of a single strain of microorganism leading, via a single fermentation process, to optimized amounts of propionic acid and vitamin B₁₂.

More specifically, the present invention relates to a fermentation process as defined in claim 1.

Unexpectedly, the claimed process leads, as a result of its two-stage organization and the choice of a strain capable of producing propionic acid and vitamin B₁₂ by fermentation and under sufficiently closely related culture conditions, to optimized yields of propionic acid and vitamin B₁₂. By its two-stage organization, the claimed process leads to the recovery, in a first stage of an optimized amount of the extracellulary formed compound i.e. the propionic acid and in a second stage of an optimized amount of the intracellulary formed compound i.e. the vitamin B₁₂. whose recovery involves the disruption of the cells.

Moreover, the claimed process is particularly useful, but not limited, for processes involving microorganisms whose growth is inhibited by the propionic acid.

Advantageously, the optimized production of propionic acid obtained according to the invention does not affect the cell growth of the microorganisms, and is hence not detrimental to the subsequent production of vitamin B₁₂.

The strain of microorganism employed according to the invention preferably belongs to the genus Propionibacterium. It makes no difference whether the strain in question is of the wild-type or otherwise. Many strains of this genus have already been the subject of description in the literature.

In the context of the claimed process, many of these strains are suitable like for example P. Shermanii PZ-3, P. Jensenii DSM 20535 and P. acidipropionici DSM 8250. Propionibacterium acidipropionici strain DSM 8250 is preferably used.

The microorganism Propionibacterium acidipropionici DSM 8250 is introduced in each of the two stages at a cell concentration of at least 50 g/l, and preferably of the order of 75 g/l, expressed as dry biomass. Naturally, these concentration values are provided only as a guide and do not constitute a limit of the field of the invention. They can, in effect, vary in accordance with the other parameters of the process (fermenter volume, nature of the microorganism, components of the nutrient medium, etc.).

The use of Propionibacterium acidipropionici strain DSM 8250 is especially advantageous.

Its use on suitable culture media, that is to say media to which traditional additives and those specific to propionic acid or vitamin B₁₂ production are added, leads, under appropriate culture conditions, to very satisfactory yields of propionic acid and vitamin B₁₂.

Acetic acid, which is usually formed during traditional processes of fermentation of propionic acid, is obtained in the present case only in small amounts. An acetic acid/propionic acid ratio of less than 0.3, or even of less than 0.2, in anaerobic conditions is obtained in the first stage.

Lastly, as an advantageous and unexpected feature, this strain of microorganism assimilates sucrose as a carbon substrate.

The use of sucrose as a carbon substrate, alone or mixed with at least one other carbon source, within the culture medium, constitutes a preferred embodiment of the claimed process.

The sucrose may be introduced as it is as a carbon source or, more preferably, in the form of molasses. The sucrose concentration in molasses varies according to the nature of the latter. Generally speaking, it varies between approximately 15 and 70 % of carbohydrate per kg of molasses.

This feature of the process is advantageous from an economic standpoint.

In effect, molasses is a raw material which is available in large amounts and is consequently inexpensive. To be able to optimize its use in the production of propionic acid and vitamin B₁₂ is of great industrial importance in respect of profitability in terms of cost.

The sucrose is preferably employed as a carbon substrate at a concentration varying roughly between 30 and 170 g/l. It is important to note that no inhibition is observed with such concentrations.

Naturally, the culture medium employed for the fermentation contains, besides sucrose, the traditional components, namely at least one assimilable nitrogen source, growth factors, mineral salts required for the growth of the said microorganism and, where appropriate, other carbon substrates.

The assimilable nitrogen source can, for example, originate from proteins extracted from cereals (wheat, maize, etc), yeasts (extract, cream of yeast), extract of corn steep, malt, peptone, ammonia, ammonium salt and/or casein.

Growth factors, for their part, are traditionally introduced into the culture medium in the form of a yeast or corn steep liquor. The use of molasses as a carbon substrate is also advantageous in this connection. As a result of its composition, it already partly supplies the nutrient medium with these growth factors.

As regards mineral salts, these are, generally speaking, ammonium sulphate, magnesium sulphate, potassium phosphate, cobalt salts, etc.

Since the Propionibacterium acidipropionici strain is not capable of producing 5,6-dimethylbenz-imidazole (DBI), it is necessary to introduce the latter into the fermentation medium for the formation of 5,6-dimethylbenzimidazolylcobamide. In the case of propionic acid production, it will be introduced at a concentration of the order of 2 mg/l. As regards, more especially, vitamin B₁₂ production, this amount will be increased to a value of the order of 10 mg/l.

In the context of the present invention, the nitrogen source can be provided by a yeast extract. Its use at a concentration of the order of 12 g/l proves especially advantageous in relation to the yield of propionic acid and/or vitamin B₁₂.

In the particular case of vitamin B₁₂ production, betaine can be also added to the culture medium, which is further enriched with cobalt salts.

Traditionally, the fermentation is carried out in the two stages of the claimed process at a pH of the order of 6.5 and a temperature of the order of 37°C or higher. Naturally, these parameters, together with the oxygenation conditions, are adjusted more precisely in each stage in relation to the product being prepared therein. More particularly, the first stage involves anaerobic conditions while the optimal production of vitamin B₁₂, in the following step, needs micro-aerobic conditions. The propionic acid and vitamin B₁₂ formed are both collected, in continuous or discontinuous fashion, during their respective stages.

Thus, during the first stage, propionic acid production is performed in a first fermenter under anaerobic conditions at a temperature of the order of 37°C and a pH of approximately 6.5. The fermenter is fed at a dilution rate of the order of 0.25 h⁻¹, and the propionic acid formed is collected continuously or discontinuously, from the said fermenter, and the fermentation medium is partly recycled, continuously or sequentially, in the second stage designed for vitamin B₁₂ production..

Advantageously, a module permitting cell recycling may be fitted to this first fermenter. A large increase in the cell density ensues. This cell recycling may be performed via an ultrafiltration module, for example. It is naturally within the capacity of a person skilled in the art to fit such a module to the fermenter and to fix the parameters for its use. Under these conditions, the propionic acid formed during the fermentation process in the fermenter of the first stage is isolated cell-free, continuously or discontinuously, from the filtration module.

By way of illustration of the present invention, a fermentation of molasses with Propionibacterium acidipropionici strain DSM 8250, under the abovementioned working conditions, with an initial biomass concentration of the order of 75 g/l and a dilution rate of the order of 0.25 h⁻¹, leads to an especially impressive hourly productivity per unit volume, since it is of the order of 3 to 5 g.l⁻¹.h⁻¹ dependent on using substrate. In addition, the fraction of acetic acid obtained according to the process of the invention proves relatively small. Thus, for the abovementioned working conditions, the acetic acid/propionic acid fraction is less than 0.3 (dependent on using substrate), against 0.45 for the traditional fermentation processes.

Naturally, these values are provided only by way of illustration of the process according to the invention, and do not constitute limits to its field of application.

Vitamin B₁₂ optimal production, for its part, is carried out according to the present invention in a second stage, following on from the first, in a second fermenter mounted in series with respect to the first fermenter. The second fermenter is fed during the fermentation process, continuously or sequentially, with fermentation medium originating from the first fermenter.

In order to optimize vitamin B₁₂ production therein, the fermentation is carried out under micro-aerobic conditions at a temperature of the order of 40°C and a pH of approximately 6.5. The culture medium employed for vitamin B₁₂ production contains, in addition, sufficient amounts of cobalt salts and dimethylbenzimidazole and optionally of betaine.

The dilution of the fermentation medium of this second fermenter is effected via the first fermenter. Naturally, the corresponding dilution rate is adjusted in accordance with the growth rate developed in the first fermenter, which growth rate is itself dependent on the consumed substrate. It is clear that such adjustments are made on the basis of the fundamental knowledge of a person skilled in the art, and constitute simple routine operations. At the end of the fermentation process, the vitamin B₁₂ is extracted according to standard techniques.

According to this embodiment, for a biomass concentration of 75 g/l with the other working parameters as mentioned above, an hourly productivity per unit volume of vitamin B₁₂ of the order of 0,4 to 1.5 mg.l⁻¹h⁻¹ is obtained dependent on using substrate.

The process which is the subject of the present invention makes it possible advantageously to obtain two compounds which are as different as vitamin B₁₂ and propionic acid in satisfactory yields and high concentrations via a continuous culture. It leads, in particular, to a yield of the order of 0.3 to 0.5 g of propionic acid per gram of carbohydrate, without consideration of intracellular produced vitamin in the first stage. Concerning the yield in vitamin B₁₂, it is more difficult to evaluate it. We assume that it is of the order of 0.2 to 0.3 mg of vitamin B₁₂ per gram of carbohydrate.

The examples presented below, without implied limitation of the present invention, will enable further advantages of the claimed process to be demonstrated.

### MATERIALS AND ANALYTICAL METHODS

### Materials:

- Fermentations are carried out in reactors equipped with stirrers and possessing a working volume of 1.5 litres. They are initiated in a discontinuous culture (batch culture) and then, after approximately 30 hours, converted into a continuous culture.
- In the case of a cell recycling at the first reactor, a sterilizable ultrafiltration module, equipped with a polysulphone ultraporous capillary column 500 µm in diameter (pore diameter 0.01 µm), is used. During this cell recycling, the contents of the reactor are pumped through the ultrafiltration module at an approximate flow rate of 40 l/h.
- A control system is fitted to check the pH, temperature and feed of substrate during the process.
- The modifications of cell densities are assessed by the optical density measured using a photometer. The control system takes account of the corresponding data.
- The pumps employed for pumping the filtrate cleared of cells to the ultrafiltration module or for pumping the fermentation medium directly to the fermenter are also controlled by the control system, and the cell concentration is adjusted accordingly.

### ANALYTICAL METHODS:

- Cell concentrations are determined by measuring the optical density at 578 nm.
- The amount of dry biomass is evaluated after centrifugation at 10,000 rpm and drying for 24 h at 80°C.
- As a carbon substrate, either sugar at a concentration of the order of 50 g/l of sucrose, or blackstrap molasses originating either from beet (45 g/l expressed as sucrose) or from cane sugar (50 g/l expressed as sucrose), or invert molasses originating from cane sugar (31 g/l expressed as carbohydrate), is used. The amounts of sucrose, glucose and fructose are determined by HPLC combined with a refractometer (distilled water was used as mobile phase). Invert molasses possesses approximately 16 % of sucrose, 27 % of glucose and 25 % of fructose per kg. Blackstrap molasses, on the other hand, contains 50 to 45 % of sucrose.
- The amounts of propionic acid and acetic acid are determined by gas chromatography using a flame ionization detector on a 2-metre column of Chromosorb 101.
- At the end of the fermentation, the bacteria are burst in 0.1 M phosphate buffer solution with 0.01 % KCN at pH 6 and for 10 minutes at 121°C. The amount of vitamin B₁₂ formed intracellularly is evaluated spectrophotometrically in dicyano form at wavelengths of 367 and 580 nm with extinction coefficients of 30.4 × 10³ and 10.2 × 10³, respectively. The isolation of the vitamin B₁₂ from the cells as well as its purification may be carried out by various methods commonly practised by a person skilled in the art and which, on that account, will not be recalled here.

In the case of the present invention, the vitamin B₁₂ is produced intracellularly in the form of 5,6-dimethylbenzimidazolylcobamide.

### PREPARATION OF THE INOCULUM

Propionibacterium acidipropionici strain DSM 8250 is used.

The storage medium contains, per litre of deionized water, 1 g of KH₂PO₄, 2 g of (NH₄)₂HPO₄, 2.5 mg of FeSO₄.7H₂O, 10 mg of MgSO₄,7H₂O, 2.5 mg of MnSO₄.H₂O, 10 mg of NaCl, 10 mg of CaCl₂.H₂O, 10 mg of CoCl₂.6H₂O, 5.0 g of yeast extract, 1.0 g of sugars and 15 g of agar. The culture is incubated on this medium at 30°C, stored at 4°C and transferred to a fresh agar medium every month. For the autoclaving operation, the pH is adjusted to a value of between 6.8 and 7.2.

The preculture medium possesses the same concentration as the storage medium. On the other hand, it does not contain agar and its sucrose concentration is increased to a value of 20 g/l.

### EXAMPLE 1

The culture is transferred from the agar medium to an Erlenmeyer with 150 ml of culture medium described above and stored at 30°C. After 48 h of storage, 150 ml of the fermentation medium are inoculated with 20 ml of the prepared preculture. The fermenter is inoculated with this preculture at a volume ratio of 15 % alter 24 hours. The compositions of the culture media employed in each of the stages of the process are described in Table I below.

**TABLE I**

| COMPOSITION OF THE CULTURE MEDIUM | 1st STAGE PRODUCTION OF PROPIONIC ACID | 2nd STAGE PRODUCTION OF VITAMIN B₁₂ |
|---|---|---|
| Yeast extract | 12 g/l | rest. |
| KH₂PO₄ | 2 g/l | rest. |
| MgSO₄.7H₂O | 200 mg/l | rest. |
| FeSO₄.7H₂O | 2.5 mg/l | rest. |
| CoCl₂.6H₂O | 20 mg/l | 100 mg/l |
| 5,6-DBI | 2 mg/l | 10 mg/l |
| pH | 6.5 | 6.5 |
| Temperature | 37°C | 40°C |
| Oxygenation | anaerobic | micro-aerobic (0.5 vvm at 100 rmp) |

All the substrates and nutrients needed for propionic acid production are introduced into the first fermenter. This first fermentation is carried out in the absence of oxygen at a pH value of 6.5 adjusted, if necessary, with 12 % aqueous ammonia solution, and at a temperature of 37°C. The propionic acid contained in the fermentation medium is recovered via an ultrafiltration module.

The process control system makes it possible to maintain a constant cell concentration and a constant working volume within the first fermenter while recovering the propionic acid formed via the filtration module, or alternatively on transferring the fermentation medium with the cells from the first fermentation stage to the second fermentation stage.

The increasing vitamin B₁₂ production in the second reactor, is resulted with an aeration of 0.5 vvm at a pH value of 6.5, which is also adjusted, where appropriate, with 6 % aqueous ammonia solution, and at a temperature of 40°C.

The productivities with respect to propionic acid and vitamin B₁₂ obtained at the end of the fermentation process are presented in Table II below.

**TABLE II**

| | BIOMASS | DILUTION | ASSAY | ASSAY g/l | PRODUCTIVITY |
|---|---|---|---|---|---|
| 1st stage | 75 g/l | 0.25 h⁻¹ | Propionic acid: 17.7 g/l | Acetic acid 5.0 | Propionic acid 4.2-4.4 g.l⁻¹.h⁻¹ |
| 2nd stage | 75 g/l | 0.03 h⁻¹ | Vitamin B₁₂: 49 mg/l | - | Vitamin B₁₂ 1-1.5 mg.l⁻¹.h⁻¹ |

### EXAMPLE 2

Advantages of cell recycling

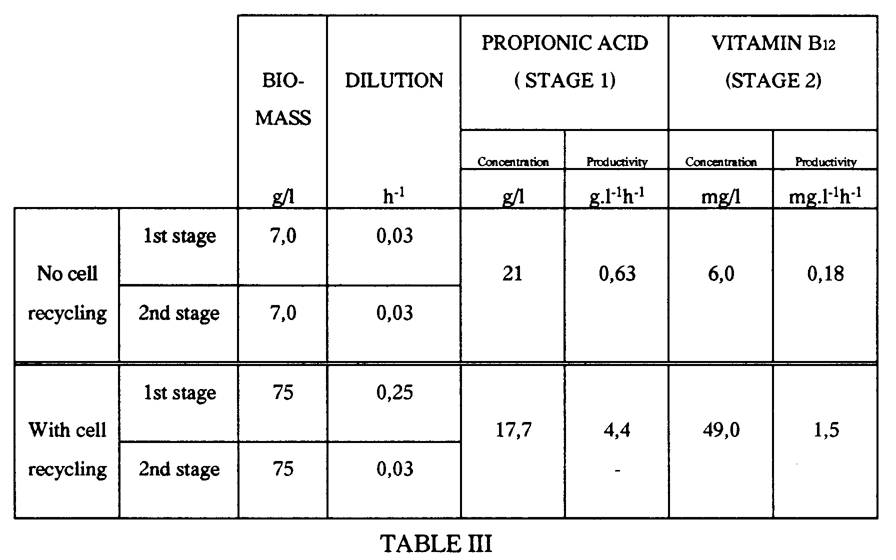

The results prove the interest of fitting to the first fermenter a module permetting cell recycling.

### EXAMPLE 3

Influence of the nature of the carbon source.

The following fermentations were performed in the two-stage process with the working conditions and parameters identified in Example 1.

**TABLE IV**

| NATURE OF THE CARBON SUBSTRATE (75 g/l) | PRODUCTIVITY WITH RESPECT TO PROPIONIC ACID (g.l⁻¹.h⁻¹) | YIELD OF PROPIONIC ACID (g/g of sucrose) | PRODUCTIVITY WITH RESPECT TO VITAMIN B₁₂ (mg.l⁻¹.h⁻¹) |
|---|---|---|---|
| Cane sugar blackstrap molasses | 4.42 | 0.5 | 1.53 |
| Beet blackstrap molasses | 4.25 | 0.5 | 1.03 |
| Invert molasses | 3.4 | 0.4 | 0.51 |

## Claims

1. Fermentation process which is useful for the simultaneous production of propionic acid and vitamin B₁₂, characterized in that it employs the culturing, on a suitable culture medium, of at least one microorganism suitable for the production of vitamin B₁₂ and propionic acid, and in that the corresponding fermentation is carried out in continuous fashion at a pH of the order of 6.5 and at a temperature of approximately 37°C or higher and involves at least two successive stages, a first stage associated with the optimal production of propionic acid said first stage being carried out under anaerobic conditions and wherein propionic acid is recovered from fermentation media, and a second with the optimal production of vitamin B₁₂ said second stage being carried out wider micro-aerobic conditions.

2. Process according to claim 1, characterized in that the microorganism employed for the fermentation belongs to the genus Propionibacterium.

3. Process according to claim 1 or 2, characterized in that the microorganism in question is Propionibacterium acidipropionici strain DSM 8250.

4. Process according to claim 3, characterized in that the microorganism Propionibacterium acidipropionici DSM 8250 is introduced in each of the two stages at a cell concentration of at least 50 g/l, and preferably of the order of 75 g/l, expressed as dry biomass.

5. Process according to one of the preceding claims, characterized in that the culture medium employs at least some sucrose, introduced as it is or in the form of molasses, as a carbon source.

6. Process according to claim 5, characterized in that the sucrose is introduced into the fermentation medium at a concentration of between 30 and 170 g/l.

7. Process according to [any] one of the preceding claims, characterized in that the step associated with propionic acid production is carried out in a first fermenter at a pH of the order of 6.5 and at a temperature of approximately 37°C.

8. Process according to claim 7, characterized in that the fermenter is fed at a dilution rate of the order of 0.25 h⁻¹.

9. Process according to any one of the preceding claims, characterized in that the propionic acid formed during the fermentation process in the fermenter of the first stage is isolated, continuously or discontinuously, from the said fermenter, and in that the fermentation medium is partly recycled, continuously or sequentially, in the second stage designed for vitamin B₁₂ production.

10. Process according to any one of the preceding claims, characterized in that a cell recycling is performed at the fermenter of the first stage.

11. Process according to claim 10, characterized in that the cell recycling is carried out via an ultrafiltration module, and in that the propionic acid is collected via the said module.

12. Process according to one of claims 1 to 11, characterized in that propionic acid is obtained in a yield of the order of 0.3 to 0.5 g of propionic acid per gram of carbohydrate.

13. Process according to any one of the preceding claims, characterized in that, at the end of the first stage, besides propionic acid, acetic acid is obtained in an acetic acid/propionic acid ratio of less than 0.3.

14. Process according to any one of the preceding claims, characterized in that the stage associated with vitamin B₁₂ production employs a second fermenter positioned in series with respect to the first fermenter, and in that fermentation is performed at a pH of the order of 6.5 and at a temperature of approximately 40°C.

15. Process according to claim 14, characterized in that the culture medium employed for vitamin B₁₂ production contains, in addition, sufficient amounts of cobalt salts and dimethylimidazole.

16. Process according to any one of the preceding claims, characterized in that the second fermenter is fed during the fermentation process, continuously or sequentially, with fermentation medium originating from the first fermenter

## Patentansprüche

1. Fermentationsverfahren, das zur gleichzeitigen Produktion von Propionsäure und Vitamin B₁₂ nützlich ist, dadurch gekennzeichnet, daß bei der Züchtung auf einem geeigneten Kulturmedium mindestens ein Mikroorganismus verwendet wird, der für die Produktion von Propionsäure und Vitamin B₁₂ geeignet ist, und daß die entsprechende Fermentation kontinuierlich bei einem pH-Wert im Bereich von 6,5 und bei einer Temperatur von etwa 37°C oder darüber durchgeführt wird und mindestens zwei aufeinanderfolgende Stufen umfaßt, eine erste Stufe im Zusammenhang mit der optimalen Produktion von Propionsäure, wobei die erste Stufe unter anaeroben Bedingungen durchgeführt wird und wobei die Propionsäure aus den Fermentationsmedien isoliert wird, und eine zweite Stufe zur optimalen Produktion von Vitamin B₁₂, wobei die zweite Stufe unter mikroaeroben Bedingungen durchgeführt wird.

2. Verfahren nach Anspruch 1, dadurch gekennzeichnet, daß der zur Fermentation verwendete Mikroorganismus der Gattung Propionibakterium angehört.

3. Verfahren nach Anspruch 1 oder 2, dadurch gekennzeichnet, daß der in Frage kommende Mikroorganismus Propionibacterium acidipropionici, Stamm DSM 8250, ist.

4. Verfahren nach Anspruch 3, dadurch gekennzeichnet, daß der Mikroorganismus Propionibacterium acidipropionici DSM 8250 in jede der zwei Stufen in einer Zellkonzentration von mindestens 50 g/l, und bevorzugt im Bereich von 75 g/l, ausgedrückt als trockene Biomasse, eingebracht wird.

5. Verfahren nach einem der vorstehenden Ansprüche, dadurch gekennzeichnet, daß im Kulturmedium mindestens etwas Saccharose, eingebracht als solche oder in Form von Melasse, als Kohlenstoffquelle enthalten ist.

6. Verfahren nach Anspruch 5, dadurch gekennzeichnet, daß die Saccharose in das Fermentationsmedium in einer Konzentration zwischen 30 und 170 g/l eingebracht wird.

7. Verfahren nach einem der vorstehenden Ansprüche, dadurch gekennzeichnet, daß die mit der Propionsäureproduktion assoziierte Stufe in einem ersten Fermenter bei einem pH-Wert im Bereich von 6,5 und bei einer Temperatur von etwa 37°C durchgeführt wird.

8. Verfahren nach Anspruch 7, dadurch gekennzeichnet, daß der Fermenter mit einer Verdünnungsrate im Bereich von 0,25 h⁻¹ beschickt wird.

9. Verfahren nach einem der vorstehenden Ansprüche, dadurch gekennzeichnet, daß die während des Fermentationsverfahrens im Fermenter in der ersten Stufe gebildete Propionsäure kontinuierlich oder diskontinuierlich aus dem Fermenter isoliert wird, und daß das Fermentationsmedium kontinuierlich oder aufeinanderfolgend in die zweiten Stufe zur Vitamin B₁₂-Produktion teilweise recycled wird.

10. Verfahren nach einem der vorstehenden Ansprüche, dadurch gekennzeichnet, daß ein Zell-Recycling im Fermenter der ersten Stufe durchgeführt wird.

11. Verfahren nach Anspruch 10, dadurch gekennzeichnet, daß das Zell-Recycling über ein Ultrafiltrationsmodul durchgeführt wird, und daß die Propionsäure über das Modul isoliert wird.

12. Verfahren nach einem der Ansprüche 1 bis 11, dadurch gekennzeichnet, daß die Propionsäure in einer Ausbeute im Bereich von 0,3 bis 0,5 g Propionsäure pro Gramm Kohlenhydrat erhalten wird.

13. Verfahren nach einem der vorstehenden Ansprüche, dadurch gekennzeichnet, daß am Ende der ersten Stufe neben Propionsäure Essigsäure in einem Essigsäure/Propionsäure-Verhältnis von weniger als 0,3 erhalten wird.

14. Verfahren nach einem der vorstehenden Ansprüche, dadurch gekennzeichnet, daß bei der mit der Vitamin B₁₂-Produktion assoziierten Stufe ein zweiter Fermenter, der in Reihe mit dem ersten Fermenter gesetzt ist, verwendet wird, und daß die Fermentation bei einem pH-Wert im Bereich von 6,5 und bei einer Temperatur von etwa 40°C durchgeführt wird.

15. Verfahren nach Anspruch 14, dadurch gekennzeichnet, daß das zur Vitamin B₁₂-Produktion verwendete Kulturmedium zusätzlich ausreichende Mengen an Kobaltsalzen und Dimethylimidazol enthält.

16. Verfahren nach einem der vorstehenden Ansprüche, dadurch gekennzeichnet, daß der zweite Fermenter während des Fermentationsverfahrens kontinuierlich oder aufeinanderfolgend mit Fermentationsmedium, das aus dem ersten Fermenter stammt, beschickt wird.

## Revendications

1. Procédé de fermentation utile pour la production simultanée d'acide propionique et de vitamine B₁₂, caractérisé en ce qu'il fait appel à la culture, sur un milieu de culture approprié, d'au moins un micro-organisme approprié pour la production de vitamine B₁₂ et d'acide propionique, et en ce que la fermentation correspondante est réalisée d'une façon continue à un pH de l'ordre de 6,5 et à une température d'environ 37°C ou plus et fait intervenir au moins deux stades successifs, un premier stade associé à la production optimale d'acide propionique, ledit premier stade étant réalisé dans des conditions anaérobies et dans lequel l'acide propionique est récupéré dans le milieu de fermentation, et un second dans lequel la production de vitamine B₁₂ est optimale, ledit second stade étant réalisé dans des conditions microaérobies.

2. Procédé selon la revendication 1, caractérisé en ce que le micro-organisme employé pour la fermentation appartient au genre *Propionibacterium*.

3. Procédé selon la revendication 1 ou 2, caractérisé en ce que le micro-organisme en question est la souche *Propionibacterium acidipropionici* DSM 8250.

4. Procédé selon la revendication 3, caractérisé en ce que le micro-organisme *Propionibacterium acidipropionici* DSM 8250 est introduit dans chacun des deux stades à une concentration en cellules d'au moins 50 g/l et de préférence de l'ordre de 75 g/l, exprimée en biomasse sèche.

5. Procédé selon l'une quelconque des revendications précédentes, caractérisé en ce que le milieu de culture emploie au moins un peu de saccharose, introduit tel quel ou sous forme de mélasse, comme source de carbone.

6. Procédé selon la revendication 5, caractérisé en ce que le saccharose est introduit dans le milieu de fermentation à une concentration comprise entre 30 et 170 g/l.

7. Procédé selon l'une quelconque des revendications précédentes, caractérisé en ce que l'étape associée à la production d'acide propionique est réalisée dans un premier fermenteur à un pH de l'ordre de 6,5 et à une température d'environ 37°C.

8. Procédé selon la revendication 7, caractérisé en ce que le fermenteur est alimenté à une vitesse de dilution de l'ordre de 0,25 h⁻¹.

9. Procédé selon l'une quelconque des revendications précédentes, caractérisé en ce que l'acide propionique formé pendant le procédé de fermentation dans le fermenteur du premier stade est isolé, en continu ou en discontinu, dudit fermenteur, et en ce que le milieu de fermentation est partiellement recyclé, en continu ou par intermittence, dans le second stade conçu pour la production de vitamine B₁₂.

10. Procédé selon l'une quelconque des revendications précédentes, caractérisé en ce qu'un recyclage des cellules est réalisé au niveau du fermenteur du premier stade

11. Procédé selon la revendication 10, caractérisé en ce que le recyclage des cellules est réalisé à l'aide d'un module d'ultrafiltration et en ce que l'acide propionique est recueilli à l'aide dudit module.

12. Procédé selon l'une quelconque des revendications 1 à 11, caractérisé en ce que l'acide propionique est obtenu avec un rendement de l'ordre de 0,3 à 0,5 g d'acide propionique par gramme de glucide.

13. Procédé selon l'une quelconque des revendications précédentes, caractérisé en ce que, à la fin du premier stade, en plus de l'acide propionique, on obtient de l'acide acétique, dans un rapport acide acétique/acide propionique inférieur à 0,3.

14. Procédé selon l'une quelconque des revendications précédentes, caractérisé en ce que le stade associé à la production de vitamine B₁₂ fait appel à un second fermenteur placé en série par rapport au premier fermenteur, et en ce que la fermentation est réalisée à un pH de l'ordre de 6,5 et à une température d'environ 40°C.

15. Procédé selon la revendication 14, caractérisé en ce que le milieu de culture employé pour la production de vitamine B₁₂ contient, en plus, des quantités suffisantes de sels de cobalt et de diméthylimidazole.

16. Procédé selon l'une quelconque des revendications précédentes, caractérisé en ce que le second fermenteur est alimenté pendant le procédé de fermentation, en continu ou par intermittence, avec le milieu de fermentation provenant du premier fermenteur.
